# EUROPÄISCHE PATENTANMELDUNG

(11) **EP 1 108 422 A2**
(43) Veröffentlichungstag der Anmeldung: **20.06.2001**
(21) Anmeldenummer: 00126753.3
(22) Anmeldetag: 06.12.2000
(51) Int. Cl.: A61K 9/00, A61K 9/06

(54) **Medizinprodukt zur Befeuchtung und Reinigung der Nasenschleimhaut**

(30) Priorität: 18.12.1999 DE 19961307
(71) Anmelder: Krewel Meuselbach GmbH, 53783 Eitorf (DE)
(72) Erfinder: Schierstedt, Dieter, Dr., 53757 St. Augustin (DE)
(74) Vertreter: Jönsson, Hans-Peter, Dr.

(57) **Zusammenfassung**

Die Erfindung betrifft ein Medizinprodukt zur Befeuchtung und Reinigung der Nasenschleimhaut, insbesondere in Form eines Nasensprays, einer Spüllösung oder Nasentropfen, das dadurch gekennzeichnet ist, dass es wasserlösliche oder in Wasser dispergierbare natürliche oder synthetische Polymere, die in wässrigen Systemen Gele oder viskose Lösungen bilden, enthält.

## Beschreibung

Die Erfindung betrifft ein Medizinprodukt zur Befeuchtung und Reinigung der Nasenschleimhaut, in Form eines Nasensprays, einer Spüllösung oder Nasentropfen.

DE 42 26 217 A1 betrifft die Verwendung von Zubereitungen zur Bekämpfung der trockenen Nase und Mittel hierfür. Die Verwendung von Zubereitungen zur Behandlung der _{"}trockenen Nase", umfassend einen oberflächenaktiven Stoff, gegebenenfalls ein Konservierungsmittel und/oder eine auf den Schleimhäuten bakterizid oder fungizid wirkende Substanz und gegebenenfalls eine die Schleimhäute geschmeidig machende Substanz, in physiologischer Kochsalzlösung, und solche Mittel werden hier beschrieben.

Das in den Ausführungsbeispielen genannte oberflächenaktive Mittel ist nicht geeignet, in wässrigen System Gele oder viskose Lösungen zu bilden.

EP 0 500 807 B1 betrifft ein Mittel zur Feuchthaltung von Gewebe. Das Verfahren umfasst insbesondere das Inkontaktbringen von Nasenschleimhaut mit einer wirksamen befeuchteten Menge einer wässrigen Zusammensetzung, die Wasser und eine befeuchtende Menge eines bioadhäsiven befeuchtenden Polymers enthält, wobei das bioadhäsive befeuchtende Polymer ein wasserquellbares, jedoch wasserunlösliches, vernetztes carboxyfuntkionelles Polymer umfasst. Somit ist das hier beschriebene Polymer nicht geeignet, in wässrigen Systeme Gele oder viskose Lösungen zu bilden.

EP 0 216 917 B1 betrifft ein therapeutisches Präparat zur nasalen Verabreichung, das unter anderem ein Andickungsmittel enthält. Als Andickungsmittel wird beispielsweise Methylcellulose genannt. US 5,843,881 A betrifft eine Sprayzusammensetzung. Die Zusammensetzung enthält insbesondere Alkohol, ein Polymer und einen Alkoholmaskierenden Parfumzusatz. Die Zusammensetzungen werden auf die Haut, das Haar oder die Schleimhaut aufgetragen.

Es ist bisher üblich, zur Behandlung des vulgären Schnupfens (Rhinitis catarrhalis), Heuschnupfens und anderen Rhinitiden Sympathomimetica lokal in Form von Nasentropfen oder Nasensprays anzuwenden, um eine Schleimhautabschwellung zu erreichen. Sympathomimetica besitzen jedoch zahlreiche unerwünschte Nebenwirkungen und können infolge einer Resorption zu Herzklopfen und Atemstörungen führen. Darüber hinaus verursachen sie die Austrocknung der Nasenschleimhaut und bei längerer Anwendung kommt es zu einer dauerhaften Nasenschleimhaut-Epithel-Schädigung, die als Rhinopathia medicamentosa bekannt ist (Apotheker-Journal 12, 30 - 34 (1985) Otto Hoffmanns Verlag, München).

Als Alternative zu den vorgenannten Zubereitungen haben sich in den letzten Jahren Sympathomimetica-freie Nasensprays auf Basis von Meersalz und/oder Kochsalz durchgesetzt. Diese haben zwar keinen akuten pharmakologischen Effekt (Medizinprodukt) auf die Nasenschleimhaut, sind aber in der Lage durch die Befeuchtung und Reinigung der Schleimhaut, insbesondere nach mehrmaliger Anwendung, die Schleimhäute zu beruhigen und so eine Linderung der Beschwerden hervorzurufen.

Da die Verweilzeit der Lösung nur kurz ist, muss die Lösung sehr häufig appliziert werden und die Linderung reicht oft bei stärkeren Beschwerden nicht aus.

Die Aufgabe der vorliegenden Erfindung besteht demgegenüber darin, an sich im Stand der Technik bekannte Medizinprodukte, insbesondere auf der Basis von Kochsalz und/oder Meersalz, zu verbessern.

Überraschenderweise wurde gefunden, dass durch Zusatz von wasserlöslichen oder in Wasser dispergierbaren Polymeren, die in wässrigen Systemen, Gele oder viskose Lösungen bilden, eine Verlängerung der Befeuchtungszeit auf der Nasenschleimhaut erreicht wird.

Eine erste Ausführungsform der Erfindung umfasst daher ein Medizinprodukt in Form von Tropfen, Spüllösungen oder Spray zur Befeuchtung und Reinigung der Nasenschleimhaut, das dadurch gekennzeichnet ist, dass es 0,1 bis 5 Gew.-% wasserlösliche oder in Wasser dispergierbare natürliche oder synthetische Polymere enthält, die in wässrigen Systemen Gele oder viskose Lösungen bilden.

Mehrere Probanden, welche häufig an Rhinitiden, auch mit Beteiligung der Stirnhöhlen oder Nebenhöhlen, litten, applizierten das erfindungsgemäße Medizinprodukt in Form eines Sprays im täglichen Wechsel mit herkömmlichen, im Handel erhältlichen Salzsprays und kamen nach einwöchiger Anwendung zu dem Ergebnis, dass das erfindungsgemäße Spray seltener angewendet werden musste und besser die Nase freihielt.

Als Polymere im Sinne der vorliegenden Erfindung kommen beispielsweise Polymere, die ausgewählt sind aus Kollagenderivate (tierisch und pflanzlich), Polyalkylenglykolen, insbesondere Polyethylenglykolen, Polyglycerinen, Alginaten, Carrageen, Pektinen, Tragant, Gumen, insbesondere Gummi Arabicum, Cellulosederivaten, insbesondere Celluloseether und/oder Celluloseester, Polyvinylalkoholen, Polyvinylpyrrolidon und/oder Dextran in Frage.

Besonders bevorzugte Cellulosederivate im Sinne der vorliegenden Erfindung umfassen Hydroxyethylcellulose und/oder Methylhydroxypropylcellulose.

Besonders bevorzugt im Sinne der vorliegenden Erfindung ist es, wenn die Polymere in einer Menge von 0,5 bis 1,5 Gew.-% enthalten sind. Eine zu geringe Menge der Polymere verhindert eine lang andauernde Filmbildung, während eine zu große Menge der Polymere die Viskosität der Medizinprodukte unerwünscht erhöht.

Eine weitere bevorzugte Ausführungsform der vorliegenden Erfindung ist dadurch gekennzeichnet, dass das Medizinprodukt Kochsalz und/oder Meersalz als Basis enthält. Anstelle oder in Kombination damit, können auch weitere tonisierende Zusätze wie physiologische Salze, Puffer oder auch ionische oder nichtionische physiologisch verträgliche Stoffe als Basis dienen. Die Verwendung von Kochsalz oder Meersalz ist im Stand der Technik weit verbreitet. Entsprechende Mittel können dabei natürliches Meerwasser mit Spurenelementen und Mineralstoffen enthalten.

In einer weiteren Ausführungsform der vorliegenden Erfindung enthält das Medizinprodukt gegebenenfalls Wirksubstanzen, insbesondere ausgewählt aus der Gruppe Antibiotika, Corticoide, Sympathomimetica, Parasympatholytica, Antiphlogistica und/oder Chemotherapeutika. Für den Fall, dass in den Medizinprodukten entsprechende Wirksubstanzen enthalten sind, handelt es sich somit um ein Arzneimittel.

Obwohl es im Sinne der vorliegenden Erfindung nicht notwendig ist, Konservierungsmittel oder sonstige Zusatzbestandteile einzusetzen, ist es im Sinne der vorliegenden Erfindung möglich, beispielsweise Konservierungsmittel zu verwenden. Besonders bevorzugte Konservierungsmittel sind prinzipiell aus dem Stand der Technik bekannt, beispielsweise aus der EP 0 240 907 B1, auf die insoweit inhaltlich Bezug genommen wird. Konservierungsmittel im Sinne der vorliegenden Erfindung sind beispielsweise Benzalkoniumchlorid, Cetylpyridiniumchlorid, Thiomersal, Phenylquecksilbersalze, Chlorhexidinacetat, Glukonat, Chlorbutanol und/oder PHB-Ester.

Mit Hilfe von pH-Wert-Regulatoren ist es möglich, den pH-Wert der erfindungsgemäßen Medizinprodukte auf einen physiologisch verträglichen pH-Wert, möglichst nicht unter pH 6,5, einzustellen. Besonders bevorzugte pH-Wert-Regulatoren im Sinne der vorliegenden Erfindung sind Natriumhydroxid, Natriumphosphat, Natriumcitrat und andere physiologisch verträgliche Puffersysteme, beispielsweise Phosphat oder Citratpuffer. Der obengenannte Grenzwert entspricht dem für Nasenspray üblichen pH-Wert, der durch die Schleimhautverträglichkeit bestimmt wird.

Die Tonie der erfindungsgemäßen Medizinprodukte wird üblicherweise in Richtung einer leichten Hypertonie (besonders bevorzugt 400 mosmol) eingestellt. Besonders bevorzugte Tonisierungsmittel umfassen insbesondere Glukose, Sorbit, Mannit und/oder Xylit.

Die galenische Zubereitung der erfindungsgemäßen Medizinprodukte kann nach den galenischen Methoden und Regeln erfolgen, wie sie für die Herstellung von wässrigen Nasentropfen allgemein üblich sind (Sucker, H. P. Fuchs und P. Speiser: Pharmazeutische Technologie, Thieme Verlag, Stuttgart (1978)). Besonders bevorzugt im Sinne der vorliegenden Erfindung werden die Medizinprodukte in Form einer wässrigen Lösung hergestellt.

Eine weitere Ausführungsform der vorliegenden Erfindung betrifft die Verwendung der oben definierten Medizinprodukte. Diese werden besonders bevorzugt bei Nasenschleimhautreizungen eingesetzt, die hervorgerufen werden unter anderem durch trockene Heizungsluft, Hausstaub, Heuschnupfen oder Schnupfen (Rhinitis catarrhalis).

Ausführungsbeispiele:

Beispiel 1:

| | |
|---|---|
| Methocel®E15 | 1,0 g |
| NaCI | 0,9 g |
| H₂O | 100 ml |

Beispiel 2:

| | |
|---|---|
| Methocel®E15 | 1,0 g |
| NaCI | 0,9 g |
| Benzalkoniumchlorid | 0,014 g |
| H₂O ad | 100 ml |

Beispiel 3:

| | |
|---|---|
| Kollidon®25 | 1,0 g |
| NaCI | 0,9 g |
| H₂O g ad | 100 ml |

Beispiel 4:

| | |
|---|---|
| PEG 3000 | 1,3 g |
| NaCI | 0,9 g |
| Benzalkoniumchlorid | 0,014 g |
| H₂O ad | 100 ml |

Beispiel 5:

| | |
|---|---|
| Methocel®E15 | 1,0 g |
| Sorbit | 0,5 g |
| H₂O ad | 100 ml |

Beispiel 6:

| | |
|---|---|
| Methocel®E15 | 1,0 g |
| Sorbit | 0,5 g |
| Benzalkoniumchlorid | 0,014 g |
| H₂O ad | 100 ml |

## Patentansprüche

1. Medizinprodukt in Form von Tropfen, Spüllösungen oder Spray zur Befeuchtung und Reinigung der Nasenschleimhaut,
dadurch gekennzeichnet, dass
es 0,1 bis 5 Gew.-% wasserlösliche oder in Wasser dispergierbare natürliche oder synthetische Polymere enthält, die in wässrigen Systemen Gele oder viskose Lösungen bilden.

2. Medizinprodukte nach Anspruch 1, dadurch gekennzeichnet, dass die Polymere ausgewählt sind aus Kollagenderivaten, Polyalkylenglykolen, insbesondere Polyethylenglykolen, Polyglycerinen, Alginaten, Carrageen, Pektinen, Tragant, Gumen, insbesondere Gummi Arabicum, Cellulosederivaten, insbesondere Celluloseether und/oder Celluloseester, Polyvinylalkoholen, Polyvinylpyrrolidon und/oder Dextran.

3. Medizinprodukt nach Anspruch 1 oder 2, dadurch gekennzeichnet, dass es die Polymere in einer Menge von 0,5 bis 1,5 Gew. %, enthält.

4. Medizinprodukt nach einem der Ansprüche 1 bis 3, dadurch gekennzeichnet, dass es weiterhin Kochsalz und/oder Meersalz enthält.

5. Medizinprodukt nach einem der Ansprüche 1 bis 4, dadurch gekennzeichnet, dass es Wirksubstanzen ausgewählt aus der Gruppe Antibiotika, Corticoide, Sympathomimetica, Parasympatholytica, Antiphlogistica und/oder Chemotherapeutika enthält.

6. Medizinprodukt nach einem der Ansprüche 1 bis 5, dadurch gekennzeichnet, dass es weiterhin Konservierungsmittel, insbesondere Benzalkoniumchlorid, Cetylpyridiniumchlorid, Thiomersal, Phenylquecksilbersalze, Chlorhexidinacetat, Glukonat, Chlorbutanol und/oder PHB-Ester, pH-Wert-Regulatoren und/oder Tonisierungsmittel, insbesondere Zucker ausgewählt aus Glucose, Sorbit, Mannit und/oder Xylit, enthält.

7. Medizinprodukt nach einem der Ansprüche 1 bis 6 in Form einer wässrigen Lösung.

8. Verwendung eines Medizinproduktes nach einem der Ansprüche 1 bis 7, dadurch gekennzeichnet, dass man es bei Nasenschleimhautreizung hervorgerufen durch trockene Heizungsluft, Hausstaub, Heuschnupfen oder Schnupfen (Rhinitis catarrhalis) einsetzt.
